# EUROPÄISCHE PATENTANMELDUNG

(11) **EP 2 336 118 A1**
(43) Veröffentlichungstag der Anmeldung: **22.06.2011**
(21) Anmeldenummer: 10012037.7
(22) Anmeldetag: 07.10.2003
(51) Int. Cl.: C07D 333/18, H01L 51/30, C08G 61/12

(54) **Lineare organische Oligomere und diese enthaltende Schichten**

(30) Priorität: 18.10.2002 DE 10248876
(62) Teilanmeldung aus: 03022655.9
(71) Anmelder: Heraeus Clevios GmbH, 63450 Hanau (DE)
(72) Erfinder: Kirchmeyer, Stephan, Dr., 51375 Leverkusen (DE); Ponomarenko, Sergei, Dr., Moskau 107589 (RU)
(74) Vertreter: Herzog, Martin

(57) **Zusammenfassung**

Die Erfindung betrifft ein Verfahren zur Herstellung von Verbindungen der allgemeinen Formel (I), 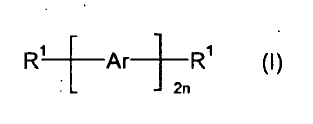 worin
n
eine ganze Zahl von 2 bis 5 ist,
R¹
für H oder eine gegebenenfalls durch ein oder mehrere O- oder S-Atome, Silylen-, Phosphonoyl- oder Phosphorylgruppen unterbrochene C₁-C₂₀- Alkylgruppe steht und
Ar
für gegebenenfalls substituiertes 1,4-Phenylen, 2,7-Fluoren oder 2,5- Thiophen steht, wobei Ar gleich oder verschieden sein kann,

halbleitende Schichten aus diesen Verbindungen sowie deren Verwendung in der Halbleitertechnik.

## Beschreibung

Die Erfindung betrifft ein Verfahren zur Herstellung linearer organischer Oligomere, halbleitende Schichten aus diesen Oligomeren sowie deren Verwendung in der Halbleitertechnik.

Das Feld molekularer Elektronik hat sich in den letzten 15 Jahren mit der Entdeckung organischer leitender und halbleitender Verbindungen rapide entwickelt. In dieser Zeit wurden eine Vielzahl von Verbindungen gefunden, die halbleitende oder elektrooptische Eigenschaften aufweisen. Es ist allgemeines Verständnis, dass die molekulare Elektronik nicht konventionelle Halbleiterbausteine auf der Basis von Silizium verdrängen wird. Stattdessen geht man davon aus, dass molekulare elektronische Bauelemente sich neue Anwendungsgebiete eröffnen werden, in denen die Eignung zur Beschichtung großer Flächen, strukturelle Flexibilität, Prozessierbarkeit bei niedrigen Temperaturen und niedrigen Kosten benötigt werden. Halbleitende organische Verbindungen werden derzeit für Anwendungsgebiete wie organische Feld-Effekt-Transistoren (OFET's), organische Lumineszenzdioden (OLED's), Sensoren und photovoltaische Elemente entwickelt. Durch einfache Strukturierung und Integration von OFET's in integrierte organische Halbleiterschaltungen werden preiswerte Lösungen für intelligente Karten (smart cards) oder Preisschilder möglich, die sich bislang mit Hilfe der Silzium-Technologie aufgrund des Preises und der mangelnden Flexibilität der Siliziumbausteine nicht realisieren lassen. Ebenfalls könnten OFET's als Schaltelemente in großflächigen flexiblen Matrixanzeigen verwendet werden.

In organischen Feldeffekt-Transistoren sind bislang zwei große Klassen von Verbindungen verwendet worden. Sämtliche Verbindungen weisen fortlaufende konjugierte Einheiten auf und werden je nach Molekulargewicht und Aufbau in konjugierte Polymere und konjugierte Oligomere unterteilt. Dabei unterscheidet man in der Regel Oligomere von Polymeren dadurch, dass Oligomere eine einheitliche molekulare Struktur und ein Molekulargewicht unter 10000 Dalton aufweisen, wohingegen Polymere in der Regel eine Molekulargewichtsverteilung aufweisen. Jedoch besteht ein fließender Übergang zwischen Oligomeren und Polymeren. Oft wird auch mit der Unterscheidung zwischen Oligomeren und Polymeren der Unterschied in der Verarbeitung dieser Verbindungen zum Ausdruck gebracht. Oligomere sind häufig verdampfbar und können über Aufdampfverfahren auf Substrate aufgebracht werden. Als Polymere werden häufig unabhängig von ihrer molekularen Struktur Verbindungen bezeichnet, die nicht mehr verdampfbar sind und daher in der Regel über andere Verfahren aufgebracht werden.

Eine wichtige Voraussetzung zur Herstellung hochwertiger organischer Halbleiterschaltungen sind Verbindungen extrem hoher Reinheit. In Halbleitern spielen Ordnungsphänomene eine große Rolle. Behinderung an einheitlicher Ausrichtung der Verbindungen und Ausprägungen von Korngrenzen führt zu einem dramatischen Abfall der Halbleitereigenschaften, so dass organische Halbleiterschaltungen, die unter Verwendung nicht extrem hochreiner Verbindungen gebaut wurden, in der Regel unbrauchbar sind. Verbleibende Verunreinigungen können beispielweise Ladungen in die halbleitende Verbindung injizieren ("Dotierung") und damit das On/Off-Ratio verkleinern oder als Ladungsfallen dienen und damit die Mobilität drastisch herabsetzen. Weiterhin können Verunreinigung die Reaktion der halbleitenden Verbindungen mit Sauerstoff initiieren und oxidierend wirkende Verunreinigungen können die halbleitenden Verbindungen oxidieren und somit mögliche Lager-, Verarbeitungs- und Betriebszeiten verkürzen.

Verdampfbare Verbindungen haben in der Regel den Vorteil, dass während des Verarbeitungsschrittes durch das Verdampfen eine weitere Reinigung erfolgt und schwer verdampfbare Verunreinigungen nicht aufgebracht werden. Verunreinigungen, die dadurch entstehen, dass neben den gewünschten Verbindungen kleine Menge anderer, aber sehr ähnlicher Verbindungen vorhanden sind, die in ähnlicher Weise verdampfen, können jedoch auf diese Weise nicht aufgetrennt werden. Somit ist es insbesondere notwendig, verdampfbare Verbindung zur Verfügung zu stellen, die derartige Verunreinigungen nicht aufweisen.

Wichtige Vertreter oligomerer halbleitender Verbindungen sind beispielsweise Oligothiophene, insbesondere solche mit endständigen Alkylsubstituenten (Adv. Mater., 2002, Band 14, S.99). Die Verwendung derartiger Oligothiophene zur Herstellung von organischen Feldeffekt-Transistoren ist beispielweise in WO-A 92/01313 und JP-A 04 133 351 beschrieben.

Es hat bereits eine ganze Reihe von Versuchen gegeben, Oligothiophene in ausreichender Reinheit herzustellen. So wird in EP-A 402 269 die Herstellung von Oligothiophenen durch oxidative Kupplung beispielweise unter Verwendung von Eisenchlorid beschrieben (S. 7, Zeilen 20-30, S. 9, Zeilen 45-55).

Die Synthesemethode führt jedoch zu Oligothiophenen, die in der kationischen Form, in der Fachwelt auch bezeichnet als dotierte Form, vorliegen (EP-A 402.269, S. 8, Zeilen 28-29). Diese Oligothiophene sind dadurch für die Anwendung in der Halbleiterelektronik unbrauchbar, da die Oligothiophene zwar in der kationischen Form gut den elektrischen Strom leiten, aber keinen Halbleitereffekt aufweisen. Zwar ist es möglich, kationische Oligothiophene z.B. durch elektrochemische oder chemische Reaktion zu reduzieren, dies ist jedoch aufwändig und führt nicht zum gewünschten Ergebnis.

Eine Alternative ist die Kupplung von lithiumorganischen Verbindungen mit Eisen(III)salzen, wie z.B. Eisen(III)chlorid. Durch diese Reaktion werden in der Regel undotierte, d.h. neutrale Oligothiophene erhalten, allerdings führen auch in dieser Reaktion Nebenreaktionen zu stark mit Eisen und Chlor verunreinigten Produkten. Statt Eisen(III)chlorid wurden andere Eisen(III)verbindungen, beispielsweise Eisen(III)acetylacetonat als Kupplungsreagenz vorgeschlagen (J. Am. Chem. Soc., 1993, 115, 12214). Aufgrund der geringeren Reaktivität dieses Kupplungsreagenzes besitzt diese Variante jedoch den Nachteil, dass die Reaktion bei erhöhter Temperatur durchgeführt werden muss. Durch die höhere Temperatur wird ein Lithium-Wasserstoff-Austausch gefördert und durch die hierdurch auftretenden Nebenreaktionen sind qualitativ hochwertige Oligothiophene auch durch intensive Reinigungsoperationen nicht zugänglich (Chem. Mater., 1995, 7, 2235).

Synthesen mit Grignard-Verbindungen (JP-A 02 250 881) oder zinkorganischen Verbindungen (US-A 5.546.889) in Anwesenheit von Nickelkatalysatoren führen ebenfalls zu Produkten, die mit hohem Aufwand gereinigt werden müssen.

Als eine weitere Möglichkeit zur Herstellung von Oligothiophenen wird in der Literatur die oxidative Kupplung durch Kupfersalze, insbesondere durch Kupfer(II)-chlorid beschrieben.

So beschreibt Kagan et al. die oxidative Kupplung von 2-Lithiothiophenen mit Kupfer(II)chlorid in Dimethylformamid/Tetrahydrofuran (Heterocycles, 1983, 20, 1937). Es wurden einige Verbesserungen in der Durchführung wie z.B. die Verwendung von komplexierten Lithiumalkylverbindungen als Lithiierungsreagentien vorgeschlagen. Jedoch wurde bei der Herstellung von beispielweise Sexithiophen gefunden, dass das Produkt nach Reinigung durch Rekristallisation weiterhin 0,77 Gew.-% Chlor und 0,033 Gew.-% Kupfer enthält, von denen zumindest das Chlor wenigstens teilweise chemisch an das Oligothiophen gebunden vorliegt und sich auch durch weitere aufwändige Reinigung nicht weiter entfernen lässt (Katz et al., Chem. Mater., 1995, 7, 2235).

Es besteht daher weiterhin Bedarf an einem verbesserten Verfahren zur Herstellung von organischen Oligomeren, insbesondere Oligothiophenen, die nur sehr geringe Mengen an Verunreinigung und somit geringe Defekte aufweisen. Insbesondere besteht Bedarf an solchen Verfahren zur Herstellung von organischen Oligomeren, bei welchen diese ohne zusätzliche aufwändige Reinigungen in hoher Qualität für die Anwendung als Halbleiter erhalten werden.

Aufgabe der vorliegenden Erfindung war es daher ein solches Verfahren bereitzustellen.

Überraschend wurde nun gefunden, dass durch Kupplung entsprechender lithiierter Ausgangsverbindungen mit Kupfer(II)chlorid organische Oligomere, insbesondere Oligothiophene, mit deutlich verbesserter Ausbeute und Qualität erhältlich sind, wenn die Kupplung mit einer Kupfer(II)verbindung so durchgeführt wird, dass die zu kuppelnde lithiumorganische Vorstufe vor Zugabe der Kupfer(II)verbindung in gelöster Form vorliegt.

Gegenstand der vorliegenden Erfindung ist daher Verfahren zur Herstellung von Verbindungen der allgemeinen Formel (I), 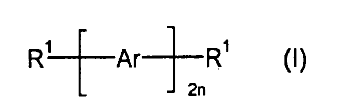 worin
- n: eine ganze Zahl von 2 bis 5, bevorzugt 2 oder 3 ist,
- R¹: für H oder eine gegebenenfalls durch ein(e) oder mehrere O- oder S-Atome, Silylen-, Phosphonoyl- oder Phosphorylgruppen unterbrochene C₁-C₂₀-Alkyl- gruppe, bevorzugt für eine C₁-C₁₂-Alkylg"ruppe steht und
- Ar: für gegebenenfalls substituiertes 1,4-Phenylen, 2,7-Fluoren oder 2,5-Thio- phen steht, wobei Ar gleich oder verschieden sein kann, bevorzugt jedoch gleich ist,
**dadurch gekennzeichnet, dass** Verbindungen der allgemeinen Formel (II), 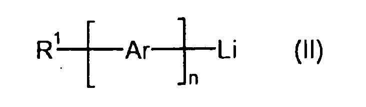 worin n, R¹ und Ar die für die allgemeine Formel (I) genannte Bedeutung haben,
bei einer Temperatur von -100°C bis +20°C in einem organischen Lösungsmittel oder Lösungsmittelgemisch vollständig gelöst und mithilfe einer oder mehrerer Kupfer(II)verbindung(en) bei Temperaturen von -100°C bis +20°C miteinander gekuppelt werden.

Als Substituenten für Ar kommen beispielsweise lineare oder verzweigte C₁-C₂₀-Alkylreste, bevorzugt C₁-C₁₂-Alkylreste, oder durch ein oder mehrere O-Atome unterbrochene lineare C₁-C₂₀-Alkylreste in Frage. An den 2,7-Fluoreneinheiten sitzen gegebenenfalls vorhandene Substituenten (einer oder mehrere) bevorzugt an der 9-Position.

Bevorzugt ist das erfindungsgemäße Verfahren ein Verfahren zur Herstellung von Verbindungen der allgemeinen Formel (I-a), 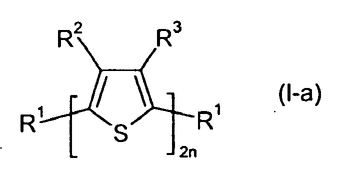 worin
- n: eine ganze Zahl von 2 bis 4, bevorzugt 2 oder 3 ist,
- R¹: für H oder eine gegebenenfalls durch ein(e) oder mehrere O- oder S-Atome, Silylen-, Phosphonoyl- oder Phosphorylgruppen unterbrochene C₁-C₂₀-Alkyl- gruppe, bevorzugt für eine C₁-C₁₂-Alkylgruppe steht und
- R², R³: unabhängig voneinander jeweils für H oder eine gegebenenfalls substituierte C₁-C₂₀-Alkylgruppe, eine gegebenenfalls substituierte C₁-C₂₀-Alkoxygruppe oder gemeinsam für eine gegebenenfalls substituierte C₁-C₆-Dioxyalkylen-gruppe, bevorzugt unabhängig voneinander jeweils für H oder eine C₁C₆-Alkylgruppe, besonders bevorzugt für H stehen,
**dadurch gekennzeichnet, dass** Verbindungen der allgemeinen Formel (II-a) 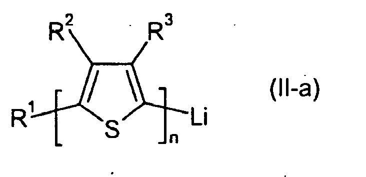 worin n, R¹, R² und R³ die für die allgemeine Formel (I-a) genannte Bedeutung haben,
bei einer Temperatur von -100°C bis +20°C in einem organischen Lösungsmittel oder Lösungsmittelgemisch vollständig gelöst und mithilfe einer oder mehrerer Kupfer(II)verbindung(en) bei Temperaturen von -100°C bis +20°C miteinander gekuppelt werden.

Als Substituenten für R² und R³ kommen in Prinzip solche in Frage, die unter den gegebenen Reaktionsbedingungen mit lithiumorganischen Verbindungen nicht reagieren, beispielsweise lineare oder verzweigte, gegebenenfalls substituierte C₁-C₂₀-Alkylreste, gegebenenfalls substituierte C₅-C₁₂-Cycloalkylreste, gegebenenfalls substituierte C₆-C₁₄-Arylreste.

C₁-C₂₀-Alkylreste sind im Rahmen der Erfindung beispielsweise Methyl, Ethyl, n-oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-Dimethylpropyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Hexadecyl oder n-Octadecyl, C₅-C₁₂-Cycloalkylreste sind beispielsweise Cyclopentyl, Cyclohexyl, Cycloheptyl, Cyclooctyl, Cyclononyl oder Cyclodecyl, C₆-C₁₄-Arylreste sind beispielsweise Phenyl, o-, m-, p-Tolyl, Benzyl, 2,3-, 2,4-, 2,5-, 2,6-, 3,4-, 3,5-Xylyl, Mesityl oder Naphthyl. Die vorangehende Aufzählung dient der beispielhaften Erläuterung der Erfindung und ist nicht als abschließend zu betrachten.

Als organische Lösungsmittel kommen im Prinzip sämtliche Lösungsmittel oder Lösungsmittelgemische in Frage, welche mit lithiumorganischen Verbindungen, wie beispielsweise solchen der allgemeinen Formel (II) oder (II-a) oder weiteren in dieser Anmeldung aufgeführten, nicht reagieren. Dies sind in der Regel Verbindungen, welche keine Halogenatome oder gegenüber lithiumorganischen Verbindungen reaktiven Wasserstoffatome aufweisen. Geeignete Lösemittel sind beispielsweise Alkane, wie z.B. Pentan, Hexan und Heptan, Aromaten, wie z.B. Benzol, Toluol und Xylole, sowie Ethergruppen enthaltende Verbindungen, wie z.B. Diethylether, tert.-Butylmethylether, Dioxan und Tetrahydrofuran. Bevorzugt werden in dem erfindungsgemäßen Verfahren Lösemittel eingesetzt, die Ethergruppen enthalten. Ganz besonders bevorzugt ist Tetrahydrofuran. Es ist allerdings auch möglich, als Lösungsmittel Mischungen aus zwei oder mehreren dieser Lösungsmittel einzusetzen. Beispielsweise können Mischungen aus dem bevorzugt verwendeten Lösungsmittel Tetrahydrofuran und Alkanen, z.B. Hexan (z.B. enthalten in kommerziell erhältlichen Lösungen von Ausgangsprodukten wie lithiumorganischen Verbindungen), verwendet werden. Wichtig im Sinne der Erfindung ist, dass das Lösungsmittel, die Lösungsmittel oder deren Gemische so gewählt werden, dass vor Zugabe der Kupfer(II)verbindung die Verbindungen der allgemeinen Formel (II) oder (II-a) in gelöster Form vorliegt.

Als Kupfer(II)verbindungen können im erfindungsgemäßen Verfahren beispielsweise Kupfer(II)halogenide, bevorzugt Kupfer(II)chlorid, Kupfer(II)bromid oder Kupfer(II)iodid, oder solche, die in den vorgenannten Lösungsmitteln zumindest teilweise löslich sind, wie beispielsweise ein Kupfer(II)salz einer Carbonsäure oder Sulfonsäure, bevorzugt Kupfer(II)acetat, Kupfer(II)citrat, Kupfer(II)acetylacetonat, Kupfer(II)glycinat, Kupfer(II)methylsulfonat, Kupfer(II)trifluormethansulfonat oder Kupfer(II)toluolsulfonat, oder ein Kupfer(II)alkoholat, bevorzugt Kupfer(II)methylat, Kupfer(II)ethylat, oder Mischungen aus diesen eingesetzt werden. Bevorzugt wird Kupfer(II)chlorid eingesetzt.

Die Kupfer(II)verbindungen werden im erfindungsgemäßen Verfahren als Kupplungsreagenzien eingesetzt. Sie werden der Reaktionslösung in kristalliner, gelöster oder teilweise gelöster Form zugegeben. Sie werden vorzugsweise in ihrer wasserfreien Form eingesetzt. Die Zugabe kann nach und nach in mehreren Portionen oder in einer Portion erfolgen. Kupfer(II)chlorid wird bevorzugt in einer Portion in kristalliner wasserfreier Form zugegeben. Die Kupfer(II)verbindungen können sowohl im äquimolaren Verhältnis zu den Verbindungen der allgemeinen Formel (II) oder (II-a) oder im Überschuss eingesetzt werden.

Die Verbindungen der allgemeinen Formel (II) oder (II-a) können entweder in isolierter Form eingesetzt oder direkt in der Reaktionslösung ("in situ") hergestellt und ohne weitere Aufarbeitung eingesetzt werden. Die "in situ"-Herstellung ist aus wirtschaftlichen Gründen bevorzugt.

Eine bevorzugte Ausführungsform des erfindungsgemäßen Verfahrens ist daher ein solches, bei dem die Verbindungen der allgemeinen Formel (II) durch Umsetzung von Verbindungen der allgemeinen Formel (III), 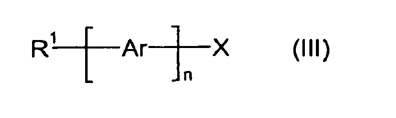 worin n, R¹ und Ar die oben genannte Bedeutung haben und
- X: für H, Cl, Br oder I steht,
mit einer lithiumorganischen Verbindung bei einer Temperatur von -100°C bis +20°C in einem organischen Lösungsmittel hergestellt werden,
wobei, gegebenenfalls bei oder nach Erwärmen auf eine Temperatur von -20°C bis +40°C, nachgerührt und anschließend wieder auf eine Temperatur von -100°C bis +20°C abgekühlt wird und
ohne weitere Aufarbeitung die Kupfer(II)verbindung zugegeben wird.

Bei der Durchführung der vorangehend beschriebenen bevorzugten Ausfiihrungsform ist es auch möglich zunächst bei einer Temperatur von -100°C bis +20°C nachzurühren, auf eine Temperatur von -20°C bis +40°C zu erwärmen und anschließend wieder auf eine Temperatur von -100°C bis +20°C abzukühlen. Die Überschneidung der Temperaturbereiche in der vorangehend beschriebenen bevorzugten Ausführungsform ist so zu verstehen, dass um auf eine bestimmte Temperatur erwärmen zu können vorher die Temperatur niedriger als diese gewesen sein muss. Diese Maßgabe gilt für jede Temperatur des überschneidenden Bereiches zwischen "von -100°C bis +20°C" und "von -20°C bis +40°C", also für jede Temperatur im Bereich von -20°C bis +20°C.

In einer besonders bevorzugten Ausführungsform des erfindungsgemäßen Verfahrens sind die Verbindungen der allgemeinen Formel (III) Verbindungen der allgemeinen Formel (III-a) 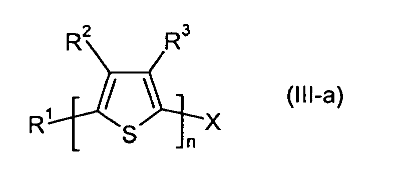 worin n, R¹, R² und R³ die oben genannte Bedeutung haben und
- X: für H, Cl, Br oder I, bevorzugt jedoch für H steht.

Geeignete lithiumorganische Verbindungen sind beispielsweise lithiumorganische Verbindung wie Lithiumalkylverbindungen, z.B. n- oder tert.-Butyllithium, Methlyllithium oder Lithiumverbindungen mit anderen Alkylresten, welche gegebenenfalls vor der Reaktion modifiziert werden, um ihre Reaktivität herabzusetzen und Nebenreaktionen, wie beispielsweise Alkylierungen zu unterdrücken. Eine solche Modifizierung kann beispielsweise durch Umsetzung der Lithiumalkylverbindungen mit Aminen erreicht werden, wobei Lithiumamide entstehen. Hierfür geeignete Amine enthalten zumindest eine Wasserstoff-Stickstoff-Bindung. Dies sind beispielsweise. Dimethylamin, Diethylamin, Dipropylamin, Diisopropylamin, Dibutylamin, Methylethylamin, Methylpropylamin, Methylbutylamin, Ethylpropylamin und Ethylbutylamin. Eine weitere Möglichkeit zur Modifizierung besteht in der Komplexierung der Lithiumalkylverbindung, z.B. durch Tetramethylethylendiamin.

Bevorzugt werden Lithiumamide, bevorzugt Lithiumdiisopropylamid, oder eine gegebenenfalls komplexierte Lithiumalkylverbindung eingesetzt, bevorzugt n- oder tert-Butyllithium oder Methyllithium.

Für die Herstellung der Verbindungen der allgemeinen Formel (II) oder (II-a) eignen sich prinzipiell dieselben, vorangehend bereits aufgeführten, Lösungsmittel bzw. Lösungsmittelgemische wie für die nachfolgende Kupplungsreaktion. Bei der "in situ"-Herstellung der Verbindungen der allgemeinen Formel (II) oder (II-a) ist entweder die Verwendung des gleichen Lösungsmittels oder lediglich eine Zudosierung eines oder mehrerer weiterer Lösungsmittel bevorzugt.

Die Initiierung der Kupplungsreaktion erfolgt bei Temperaturen von -100°C bis +20°C, vorzugsweise bei -80°C bis -60°C durch Zugabe der Kupfer(II)verbindung. Nach Zugabe der Kupfer(II)verbindung wird beispielsweise für einen Zeitraum von 5 Minuten bis 5 Stunden, gegebenenfalls auch länger, bei einer Temperatur von -100°C bis +20°C gerührt. Danach kann es notwendig sein, die Kupplungsreaktion durch Nachreaktion bei Temperaturen von -80°C bis +40°C zu vervollständigen. Beispielsweise kann dazu die Temperatur des Reaktionsansatzes zunächst langsam gesteigert werden und durch Nachrühren bei einer höheren Temperatur die Reaktion schließlich vervollständigt werden. Die Zeit welche die Kupplungsreaktion zur Vervollständigung benötigt, ist von der gewählten Temperatur abhängig. Die Vollständigkeit der Reaktion kann durch einfache Methoden, beispielsweise Dünnschichtchromatographie überprüft werden. Bei geeigneter Wahl der Reaktionsbedingungen verläuft die Kupplungsreaktion weitgehend vollständig. Weitgehend vollständig ist im Rahmen der Erfindung die Kupplungsreaktion dann, wenn kein weiterer Umsatz mehr beobachtet werden kann. Dies ist unabhängig von der umgesetzten Stoffmenge der Ausgangsverbindungen.

Die Aufarbeitung der Reaktionsmischung erfolgt nach an sich bekannten Verfahren, z.B. durch Verdünnen, Fällen, Filtration, Extraktion, Waschen, Rekristallisation aus geeigneten Lösungsmitteln, Chromatographie und/oder Sublimation. Beispielsweise kann eine Aufarbeitung in der Weise erfolgen, dass die Reaktionsmischung nach vervollständigter Reaktion in ein Gemisch aus saurem Wasser oder Eiswasser, z.B. hergestellt aus Wasser oder Eiswasser und 1 M HCl im Volumenverhältnis 20:1, und Diethylether gegossen wird, die organische Phase abgetrennt, mit Wasser gewaschen, das als Feststoff enthaltene Produkt abfiltriert, mit Diethylether gewaschen und anschließend im Vakuum getrocknet wird. Die Verbindungen der allgemeinen Formel (I) oder (I-a) sind halbleitend und können bereits ohne weitere anschließende Reinigungsprozesse in hoher Qualität und Reinheit erhalten werden und enthalten nur geringe Mengen an Verunreinigungen, wie z.B. 0,5 Gew.-% Chlor und weniger, bevorzugt 0,3 Gew.-% Chlor und weniger, besonders bevorzugt sogar 0,03 Gew.-% Chlor und weniger und/oder 0,1 Gew.-% Kupfer und weniger, bevorzugt sogar 0,02 Gew.-% Kupfer und weniger. Es ist jedoch möglich, diese Produkte nach bekannten Verfahren, z.B. durch Rekristallisation, Chromatographie oder Sublimation weiter zu reinigen.

Mit dem erfindungsgemäßen Verfahren können somit erstmals ohne aufwändige Reinigungsverfahren organische Oligomere, unter welchen im Rahmen der Erfindung Verbindungen der allgemeinen Formel (I) oder (I-a) zu verstehen sind, insbesondere Oligothiophene, mit nur sehr geringen Mengen an Verunreinigungen und somit geringen Defekten hergestellt werden. Überraschend zeigt das erfindungsgemäße Verfahren nicht die Nachteile des von Kagan beschriebenen Verfahrens (Heterocycles, 1983, 20, 1937), nämlich das trotz Reinigung durch Rekristallisation weiterhin 0,77 Gew.-% Chlor als Verunreinigung enthalten sind, von denen das Chlor wenigstens teilweise chemisch an das Oligothiophen gebunden vorliegt und sich auch durch weitere aufwändige Reinigung nicht weiter entfernen lässt (Chem. Mater., 1995,7,2235).

Ein weiterer Vorteil des erfindungsgemäßen Verfahrens gegenüber Kagan et al. ist der, dass eine "in situ"-Herstellung insbesondere der bevorzugten Verbindungen der allgemeinen Formel (II) oder (II-a) möglich ist, da kein Überschuss der Verbindungen der allgemeinen Formel (III) oder (III-a) erforderlich ist. Bei Kagan et al. wird ein zweifacher Überschuss an Thiophen verwendet, da ansonsten die Bildung erheblicher Mengen an ungewünschten Nebenprodukten (z.B. Dimeren) beobachtet wird, welche nur schwer vom gewünschten Produkt zu trennen sind. Das überschüssige Thiophen muss jedoch vor der Kupplung entfernt werden, was nicht nur zusätzlichen Aufwand bedeutet, sondern zudem zu einem Verlust von 15 % des im Überschuss eingesetzten Thiophens führt (Heterocycles, 1983, 20, 1937).

Des Weiteren können die Verbindungen der allgemeinen Formel (I) oder (I-a) nach dem erfindungsgemäßen Verfahren weitgehend frei von Oligomeren mit höherem oder niedrigerem Molekulargewicht hergestellt werden, womit die aufwändige Aufreinigung schwer trennbarer Gemische entfällt.

Die nach dem erfindungsgemäßen Verfahren hergestellten Verbindungen der allgemeinen Formel (I) oder (I-a) sind neutral und halbleitend und eignen sich aufgrund ihrer Reinheit besonders gut für den Einsatz als Halbleiter in aktiven und lichtemittierenden elektronischen Bauelementen wie Feldeffekt-Transistoren, organischen Lumineszenzdioden, photovoltaischen Zellen, Lasern oder Sensoren. Zur Verwendung werden die erfindungsgemäß hergestellten Verbindungen der allgemeinen Formel (I) oder (I-a) in Form von Schichten auf geeignete Substrate, zum Beispiel auf mit elektrischen oder elektronischen Strukturen versehene Silizium-Wafer, Polymerfolien oder Glasscheiben aufgetragen. Für den Auftrag kommen prinzipiell sämtliche dem Fachmann bekannten Auftragsverfahren in Betracht. Beispielsweise können die Verbindungen der allgemeinen Formel (I) oder (I-a) aus Lösung aufgetragen werden, wobei man das Lösungsmittel anschließend verdampft. Das Aufbringen aus Lösung kann nach den bekannten Verfahren, beispielsweise durch Sprühen, Tauchen, Drucken und Rakeln, Spin-Coating und durch Ink-Jet-Druck erfolgen. Die Verbindungen der allgemeinen Formel (I) oder (I-a) können ebenfalls aus der Gasphase, z.B. durch Aufdampfen, aufgebracht werden.

Gegenstand der vorliegenden Erfindung sind daher weiterhin Schichten enthaltend, bevorzugt im Wesentlichen bestehend aus, Verbindungen der allgemeinen Formel (I) 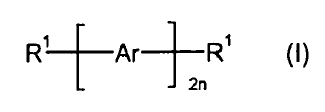 worin n, R¹ und A die oben genannte Bedeutung haben
**dadurch gekennzeichnet, dass** sie 0,5 Gew.-% Chlor und weniger, bevorzugt 0,3 Gew.-% Chlor und weniger, besonders bevorzugt 0,03 Gew.-% Chlor und weniger enthalten und halbleitend sind.

Unter 0,5 Gew.-% Chlor und weniger, 0,3 Gew.-% Chlor und weniger bzw. 0,03 Gew.-% Chlor und weniger sind im Sinne der Erfindung 0 Gew.-% bis 0,5 Gew.-% Chlor, 0 Gew.-% bis 0,3 Gew.-% Chlor bzw. 0 Gew.-% bis 0,03 Gew.-% zu verstehen. Die gegebenenfalls vorhandenen Chlorverunreinigungen können sowohl chemisch gebundenes Chlor als auch nicht chemisch gebundenes Chlor beispielsweise in Form von Chlorverbindungen, wie z.B. Chlorsalzen, sein.

Bevorzugt sind dies Schichten, worin die Verbindungen der allgemeinen Formel (I) solche der allgemeinen Formel (I-a) sind, 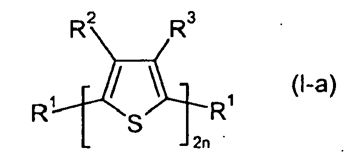 worin n, R¹, R² und R³ die oben genannte Bedeutung haben.

In besonders bevorzugten Ausführungsformen sind dies Schichten, welche für die Verwendung in aktiven und lichtemittierenden elektronischen Bauelementen wie Feldeffekt-Transistoren, organischen Lumineszenzdioden, photovoltaischen Zellen, Lasern oder Sensoren geeignet sind.

Die erfindungsgemäßen Schichten können nach dem Aufbringen weiter modifiziert werden, beispielsweise durch eine Temperaturbehandlung, z.B. unter Durchlaufen einer flüssigkristallinen Phase, oder zur Strukturierung z.B. durch Laserablation.

### Beispiele

5-Hexyl-2,2':5',2"-terthiophene und 5-Ethyl-2,2':5',2"-terthiophene wurden nach bekannten Verfahren hergestellt (Synthesis, 1993, S.1099; Chem. Mater., 1993, Band 5, S.430).

Alle Reaktionsgefäße wurden vor Gebrauch nach üblicher Schutzgastechnik ausgeheizt und mit Stickstoff geflutet.

### Beispiel 1:

Herstellung von 5,5'""-Dihexyl-2,2':5',2":5",2"':5'",2"":5"",2"'"-sexithiophene (I-a-1) 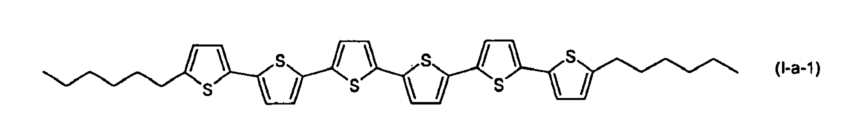 1.2 ml n-Butyllithium (BuLi, 2.5 molare (M) Lösung in Hexan, 3 mmol) wurden bei -78°C unter Stickstoff zu 10 ml absolutiertem Tetrahydrofuran (abs. thf) gegeben. Anschließend wurde eine Lösung aus 334 mg Diisopropylamin (3.3 mmol) in 5 ml abs. thf während 5 Minuten (min) zugegeben und weitere 5 min nachgerührt. Daraufhin wurde eine Lösung aus 1.0 g 5-Hexyl-2,2':5',2"-terthiophen (3 mmol) in 20 ml abs. thf innerhalb von 20 min zugetropft, wonach in der gelben klaren Lösung kein Niederschlag bzw. keine Trübung zu erkennen war. Die Reaktionslösung wurde 30 min bei -78°C gerührt und anschließend auf 0°C kommen gelassen. Nach erneutem Abkühlen auf -78°C wurden 404 mg CuCl₂ (3 mmol) in einer Portion zu der klaren, gelben Lösung gegeben und für eine Stunde bei -78°C gerührt. Die Temperatur der Reaktionsmischung wurde nach Entfernen des Kältebades (Aceton/Trockeneis) langsam auf Raumtemperatur (23°C) kommen gelassen. Nach weiteren 16 h Rühren bei Raumtemperatur wurde die Reaktionsmischung in 200 ml Diethylether gegossen und eine Mischung aus 200 ml entionisiertem (dest.) Wasser und 10 ml 1 M HCl zugegeben. Die organische Phase, die orangefarbenen Niederschlag enthielt, wurde abgetrennt, mit dest. Wasser gewaschen und filtriert. Der abfiltrierte Niederschlag wurde mit abs. Diethylether gewaschen und im Vakuum getrocknet. Es wurden 605 mg (61 % der Theorie) des Produktes in Form eines orangefarbenen Pulvers erhalten.
Das Produkt enthielt gemäß Elementaranalyse 0.02 Gew.-% Chlor und 0,014 Gew.-% Kupfer.
FD MS Analyse: M^{•+} 100%, m/e = 662.2
Schmelzverhalten (°C): K 297 SmX 304 N 312 I (K = kristallin, SmX = smektisch flüssigkristallin, N = nematisch flüssigkristallin, I = isotrop flüssig; die Zahlenwerte zwischen den Phasenbezeichnungen geben die Übergangstemperatur in °C an, z.B. K 297 SmX = Übergang von der kristallinen zur smektisch flüssigkristallinen Phase bei 297°C)
(Schmelzverhalten bestimmt mit DSC (Differential Scanning Calorimeter) Mettler TA-4000 Thermosystem, Scanning rate 1K/min)

### Beispiel 2:

Herstellung von 5,5""'-Diethyl-2,2':5',2":5",2"':5"',2"":5"",2""'-sexithiophen (I-a-2) 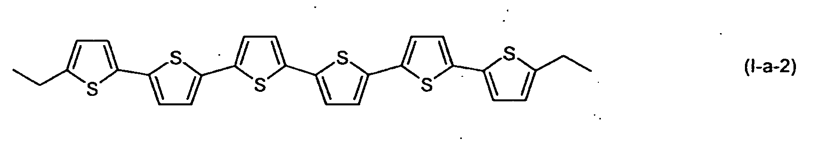 0.8 ml n-Butyllithium (BuLi, 2.5 M Lösung in Hexan, 2 mmol) wurden bei -78°C unter Stickstoff zu 10 ml absolutiertem Tetrahydrofuran (abs. thf) gegeben. Anschließend wurde eine Lösung aus 223 mg Diisopropylamin (2.2 mmol) in 5 ml abs. thf während 5 Minuten (min) tropfenweise zugegeben und weitere 5 min bei der gegebenen Temperatur nachgerührt. Daraufhin wurde eine Lösung aus 553 mg 5-Ethyl-2,2':5',2"-terthiophen (2 mmol) in 20 ml abs. thf innerhalb von 10 min zugetropft, wonach in der gelben klaren Lösung kein Niederschlag bzw. keine Trübung zu erkennen war. Die Reaktionslösung wurde 30 min bei -75°C gerührt und anschließend auf 0°C kommen gelassen. Nach erneutem Abkühlen auf -78°C wurden 538 mg CuCl₂ (4 mmol) in einer Portion zu der klaren, gelben Lösung gegeben und für 10 min bei -78°C gerührt. Die Temperatur der Reaktionsmischung wurde nach Entfernen des Kältebades (Aceton/Trockeneis) langsam auf Raumtemperatur (23°C) kommen gelassen. Nach einer weiteren Stunde Rühren bei Raumtemperatur wurde die Reaktionsmischung in 200 ml Diethylether und eine Mischung aus 200 ml Eiswasser und 10 ml 1 M HCl gegossen. Die organische Phase, die orangefarbenen Niederschlag enthielt, wurde abgetrennt, mit dest. Wasser gewaschen und filtriert. Der abfiltrierte Niederschlag wurde mit abs. Diethylether gewaschen und im Vakuum bis zur Gewichtskonstanz getrocknet. Es wurden 422 mg (77 % der Theorie) des Produktes in Form eines orangefarbenen Pulvers erhalten.
Das Produkt enthielt gemäß Elementaranalyse 0.13-0.14 Gew.-% Chlor.
FD MS Analyse: M^{•+} 100%, m/e = 550.1
Schmelzverhalten (°C): K 300 I (K = kristallin, I = isotrop flüssig)

## Patentansprüche

1. Schichten enthaltend Verbindungen der allgemeinen Formel (I) 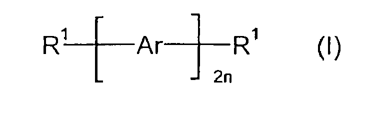 worin
n eine ganze Zahl von 2 bis 5 ist,
R¹ für H oder eine gegebenenfalls durch ein(e) oder mehrere O- oder S-Atome, Silylen-, Phosphonoyl- oder Phosphorylgruppen unter- brochene C₁-C₂₀-Alkylgruppe steht und
Ar für gegebenenfalls substituiertes 1,4-Phenylen, 2,7-Fluoren oder 2,5- Thiophen steht, wobei Ar gleich oder verschieden sein kann,
**dadurch gekennzeichnet, dass** sie 0,5 Gew.-% Chlor und weniger enthalten und halbleitend sind.

2. Schichten gemäß Anspruch 1, **dadurch gekennzeichnet, dass** sie 0,3 Gew.-% Chlor und weniger enthalten.

3. Schichten gemäß Anspruch 1 oder 2, **dadurch gekennzeichnet, dass** die Verbindungen der allgemeinen Formel (I) solche der allgemeinen Formel (I-a) sind, 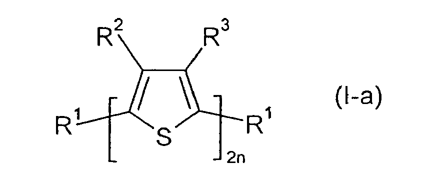 worin n, R¹ die in Anspruch 1 genannte Bedeutung haben, und
R², R³ unabhängig voneinander jeweils für H oder eine gegebenenfalls substituierte C₁-C₂₀-Alkylgruppe, eine gegebenenfalls substituierte C₁-C₂₀-Alkoxygruppe oder gemeinsam für eine gegebenenfalls substituierte C₁-C₆-Dioxyalkylengruppe stehen.

4. Verwendung der Schichten gemäß wenigstens einem der Ansprüche 1 bis 3 als Halbleiter in aktiven und lichtemittierenden elektronischen Bauelementen wie Feldeffekt-Transistoren, organischen Lumineszenzdioden, photovoltaischen Zellen, Lasern oder Sensoren.

5. Verfahren zur Herstellung der Schichten gemäß wenigstens einem der Ansprüche 1 bis 3 **dadurch gekennzeichnet, dass** Verbindungen der allgemeinen Formel (I), 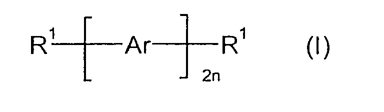 worin n, R¹ und Ar die in wenigstens einem der Ansprüche 1 bis 3 genannte
Bedeutung haben,
aus Lösung oder aus der Gasphase auf ein geeignetes Substrat aufgebracht werden.

6. Verfahren gemäß Anspruch 5, **dadurch gekennzeichnet, dass** die Verbindungen der allgemeinen Formel (I) aus der Gasphase aufgebracht werden.

7. Verbindungen der allgemeinen Formel (I) 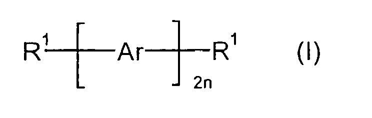 worin n, R¹ und Ar die in wenigstens einem der Ansprüche 1 bis 4 genannte
Bedeutung haben
**dadurch gekennzeichnet, dass** sie 0,5 Gew.-% Chlor und weniger enthalten.
